# EUROPÄISCHE PATENTSCHRIFT

(11) **EP 3 528 745 B1**
(45) Veröffentlichungstag und Bekanntmachung des Hinweises auf die Patenterteilung: **29.07.2020**
(21) Anmeldenummer: 17811822.0
(22) Anmeldetag: 18.10.2017
(51) Int. Cl.: A61D 19/02

(54) **VORRICHTUNG FÜR DIE KÜNSTLICHE BESAMUNG EINES SÄUGETIERES**
DEVICE FOR ARTIFICIALLY INSEMINATING A MAMMAL
DISPOSITIF D'INSÉMINATION ARTIFICIELLE D'UN MAMMIFÈRE

(30) Priorität: 18.10.2016 AT 4802016
(43) Veröffentlichungstag der Anmeldung: 28.08.2019
(73) Patentinhaber: Smartbow GmbH, 4675 Weibern (AT)
(72) Erfinder: AUER, Wolfgang, 4675 Weibern (AT)
(74) Vertreter: Mannion, Sally Kim
(86) Internationale Anmeldenummer: PCT/AT2017/000072
(87) Internationale Veröffentlichungsnummer: WO 2018/071929

(56) Entgegenhaltungen:
- EP-A1- 2 215 992
- CN-U- 203 841 849
- US-A- 2 942 603

## Beschreibung

Die Erfindung betrifft eine Vorrichtung für die künstliche Besamung eines Säugetieres wie typischerweise einer Kuh.

Bei der künstlichen Besamung einer Kuh, wird Sperma eines Stieres instrumentell in den Uterus der empfängnisbereiten Kuh eingebracht, um bestimmungsgemäß dort die Befruchtung einer Eizelle der Kuh zu bewirken und somit ein Jungtier zu zeugen. Der wesentliche Grund künstliche Besamung anstatt natürlicher Begattung zu wählen ist, dass es damit besser möglich ist, ein für den jeweiligen Zuchtzweck optimales Vatertier, welches auch weit entfernt leben kann, auszuwählen.

Die künstliche Besamung einer Kuh erfolgt zurzeit typischerweise folgendermaßen:
Schon im Vorfeld wurde anhand des angestrebten Zuchtzieles ausgewählt, von welchem Stier das zu verwendende Sperma stammen soll. Das für den Transport in gefrorenem Zustand mit speziellen Stoffen verdünnte Sperma ist für den Transport und die Handhabung in einer sogenannten Paillette, typischerweise als strohhalmartiges dünnes, beidseits verschlossenes Röhrchen ausgeführt, aufbewahrt. Der Inhalt einer Paillette liegt typischerweise bei 0,25 bis 0,5 ml. Beim typischen Transport vom Stier zur Kuh befinden sich mehrere Pailletten umgeben von flüssigem - also -196°C kalten - Stickstoff in einem wärmeisolierenden Gefäß. Zur künstlichen Besamung entnimmt ein Besamungstechniker die passende Paillette dem wärmeisolierenden Gefäß mit Flüssigstickstoff und erwärmt die Paillette typischerweise in 37°C warmem Wasser ca. 30 Sekunden etwa auf Körpertemperatur. Durch das Auftauen des in der Paillette enthaltenen Spermas werden die Samen wieder aktiv. Im nächsten Schritt wird die Paillette in die Besamungsspritze (oftmals auch "Besamungspistole" genannt) eingesetzt. Die Besamungsspritze ist im Wesentlichen ein schlankes Rohr innerhalb dessen ein dünner Stab als Kolben längsgeführt bewegbar ist. Zum Einsetzen der Paillette in die Besamungsspritze wird der Kolben an der Vorderseite der Besamungsspritze etwas hinausgeschoben, dann die Paillette mit einem Ende auf den Kolben aufgesteckt, dann der Kolben mitsamt der Paillette soweit in das Rohr zurückgezogen, dass die Paillette im Rohr verschwindet. Wenn nach dem Einsetzen der Paillette in die Besamungsspritze durch den Besamungstechniker noch etwas Weg bis zur Kuh zurückzulegen ist, wird die Besamungsspritze typischerweise auf Körpertemperatur gehalten, indem sie der Besamungstechniker an seinem Körper, unter seiner Kleidung hält. Im nächsten Schritt wird die Besamungsspritze mit der Vorderseite voran in eine sogenannte Besamungshülle, das ist im Wesentlichen ein steriles, nur einmal zu verwendendes Stück Kunststoffschlauch eingesteckt, bis die Besamungsspritze mit ihrer Vorderseite an der bis auf eine kleine Öffnung verschlossenen Stirnseite der Besamungshülle von innen her anliegt.

Anschließend wird die durch die Besamungshülle umschlossene Besamungsspritze mit der Vorderseite voran in die Scheide der Kuh eingeführt. Der Vorgang erfordert großes Geschick des Besamungstechnikers. Während er mit einer Hand die Besamungsspritze hält und bewegt, befindet sich seine zweite Hand im Rektum der Kuh, um mit den Fingerspitzen durch die Wand des Rektums und der Scheide hindurch die Position der Besamungsspritze zu ertasten und den Gebärmutterhals der Kuh zu ertasten und ruhig zu halten, damit die Stirnseite der Besamungsspritze in und durch den Gebärmutterhals geschoben werden kann. Idealerweise dann, wenn die Besamungsspritze etwa 0,6 bis 1,25 cm in den Uterus hineinreicht, wird die Besamungsspritze ausgelöst. Durch das Auslösen wird der Kolben in das hintere Ende der Paillette hineinverschoben, womit der Inhalt der Paillette aus dem vorderen Ende hinausgedrängt wird und durch die feine Öffnung an der Stirnseite der Besamungshülle hindurch in den Uterus der Kuh hinein gedrückt wird.

Es gibt mehrere Ansätze dazu, einzelne Arbeitsschritte dieses Arbeitsablaufes zu vereinfachen und/oder deren Fehleranfälligkeit zu verringern:
Die US 2013129331 A1 beschreibt ein tragbares Gerät unter Anderem für das Erwärmen der Pailletten mit Stiersperma. Im Wesentlichen besteht das Gerät aus einem Behältnis für Wasser und einer elektrischen Heizeinrichtung dafür, welche batteriebetrieben und temperaturgeregelt ist.

Die US6676596 B2 zeigt ein leichtgewichtiges, tragbares, temperaturgeregelt beheiztes Gehäuse für die Aufnahme, Vorwärmung und Warmhaltung einer mit einer vorerwärmten Paillette beladenen Besamungsspritze beim Transport an den Ort der Verwendung.

Die KR 20150004453 A zeigt eine Vorrichtung für das geregelte Erwärmen und Warmhalten einer beladenen oder leeren Besamungsspritze. Bestimmungsgemäß wird die Besamungsspritze in das innere Rohr der als doppelwandiges Rohr ausgeführten Vorrichtung eingesteckt. Die Vorrichtung weist im Raum zwischen den beiden Rohren eine geregelte elektrische Heizung auf, die über einen Stecker mit einer Steckdose eines Fahrzeuges verbindbar und dadurch mit elektrischer Energie versorgbar ist.

Die DE 60030361 T2 beschreibt eine Vorrichtung die bestimmungsgemäß wahlweise sowohl als Besamungsspritze verwendbar ist, als auch für die Transplantation einer befruchteten Eizelle in den Uterus eines Nutztieres.

Die Vorrichtung weist ein langes dünnes Rohr und eine darin und stirnseitig darüber hinaus bewegbare dünne Schlauchleitung auf. Gegebenenfalls ist am hinteren Ende der Schlauchleitung eine Paillette angeschlossen und ein Antriebsmittel an dem von der Schlauchleitung abgewandt liegenden Ende der Paillette. Bestimmungsgemäß wird das dünne Rohr in die Scheide bis an den Uterus des Tieres eingeführt, dann die Schlauchleitung weiter in den Uterus hinein verschoben, dann Sperma aus der Paillette hinaus und durch die gesamte Schlauchleitung hindurch in den Uterus des Tieres hineingedrückt. Der die Paillette umfassende Teil ist als Rohr aus einem wärmeisolierenden Material ausgebildet, dessen Zweck es ist, die Temperatur an der anderweitig vorerwärmten Paillette konstant zu halten und die Paillette und die daran angrenzenden Teile der Vorrichtung auch mechanisch zu schützen. Problematisch an der Vorrichtung ist, dass das in den Uterus einzubringende Sperma nach dem Verlassen der Paillette durch eine verhältnismäßig lange Schlauchleitung fließen muss. Damit ist es schwierig zu vermeiden, dass eine große Menge des Spermas letztendlich in dem Schlauch bleibt. Die Schlauchleitung kann zudem entweder nur für einen einzigen Besamungsvorgang verwendet werden, oder sie muss nach einem Besamungsvorgang sehr aufwändig gereinigt und sterilisiert werden.

Die US 2942603 A zeigt eine Besamungsspritze, welche die Form einer Pistole hat und der künstlichen Besamung von Geflügel dient. In einen rohrförmigen, elektrisch beheizbaren Hohlraum der Besamungsspritze wird flüssiges Sperma eingebracht. Durch händisches Drücken eines Auslösehebels wird ein Kolben von einem Ende her in den Hohlraum eingedrückt, wodurch das flüssige Sperma aus dem Hohlraum und ein daran angeschlossenes Rohr hinausgedrückt wird.

Nachteilig ist, dass Sperma als Verunreinigung im Hohlraum verbleibt. Damit muss die Spritze aufwendig gereinigt werden, bevor sie wieder angewandt werden kann, weil ansonsten nicht sicher ist, welches Sperma letztendlich eine Befruchtung ausgelöst hat.

Die CN 203 841 849 U offenbart eine Vorrichtung für die künstliche Besamung eines Säugetieres gemäß dem Oberbegriff des Anspruchs 1. Die der Erfindung zugrunde liegende Aufgabe besteht darin, eine Vorrichtung für die künstliche Besamung eines Säugetieres wie typischerweise einer Kuh so auszubilden, dass bei einfacher Handhabbarkeit die Sicherheit erhöht wird, dass das in den Uterus des Tieres einzubringende Sperma beim Einbringen nicht mit anderem in der Besamungsvorrichtung befindlichem Sperma vermengt wird.

Diese Aufgabe wird durch eine Vorrichtung für die künstliche Besamung eines Säugetieres gemäß Anspruch 1 gelöst. Bevorzugte Ausführungsformen sind in den Unteransprüchen definiert. Für das Lösen der Aufgabe wird von einer Bauweise ausgegangen, bei welcher die Vorrichtung für die künstliche Besamung einen beheizbaren Hohlraum aufweist, welcher beheizbar ist und in welchem eine Sperma enthaltende Paillette aufbewahrt und erwärmt und/oder auf Temperatur gehalten werden kann. Als erfindungsgemäße Verbesserung dazu wird vorgeschlagen, die Vorrichtung mit einem Mechanismus auszustatten, mit Hilfe dessen die Paillette aus dem Hohlraum hinaus in ein längliches Rohr verschiebbar ist, welches in das zu besamende Tier eingeführt wird.

Indem die Paillette in besagtem Hohlraum der Vorrichtung für die künstliche Besamung erwärmbar ist, kann der Zwischenschritt des Erwärmens einer Paillette vor dem ohnedies nötigen Einbringen in die Vorrichtung entfallen.

Indem die Paillette durch einen Teil der Vorrichtung in jenes längliche Rohr hinein verschiebbar ist, welches bestimmungsgemäß in das besamende Tier eingeführt wird, braucht das in der Paillette enthaltende Sperma nicht mit der Wand des Rohres in Kontakt zu kommen. Damit ist das Rohr auch ohne Zwischenreinigung öfter verwendbar und es können bei nacheinander folgenden Besamungsvorgängen Spermien von verschiedenen Vatertieren verwendet werden, ohne dass die Gefahr einer Vermengung oder unklaren Zuordnung besteht.

Indem die Heizung geregelt ist, können Temperatur-Zeit-Verläufe einschließlich optimaler Endtemperatur an der Paillette in einem engen Rahmen genau vorgegeben und durch die Regelung verlässlich erzwungen werden, womit Temperaturschocks für das Sperma zuverlässig vermeidbar sind.

Die geregelte Heizung ist am Besten mittels einem oder mehreren ohmschen elektrischen Heizwiderständen, einem oder mehreren Temperatursensoren, einer bevorzugt wieder aufladbaren elektrischen Batterie, und einer Steuerungseinheit einschließlich Stellelementen für den elektrischen Strom durch den bzw. die elektrischen Heizwiderstand auszubilden. Die weiteren technischen Details dazu sind im Rahmen des fachmännischen Handelns festlegbar, sodass hier nicht weiter darauf eingegangen zu werden braucht.

Die Erfindung wird an Hand von stark stilisierten Zeichnungen zu beispielhaften erfindungsgemäßen Vorrichtungen für die künstliche Besamung veranschaulicht:
- Fig. 1:: ist eine seitliche Teilschnittansicht einer ersten, einfachen Ausführung einer beispielhaften erfindungsgemäßen Vorrichtung.
- Fig. 2:: zeigt in Seitenansicht eine zweite erfindungsgemäße Vorrichtung, welche gegenüber jener von Fig. 1 mehrere zusätzliche Funktionen anbietet. Manche der verdeckten Einbauten sind durch strichlierte Linien dargestellt.

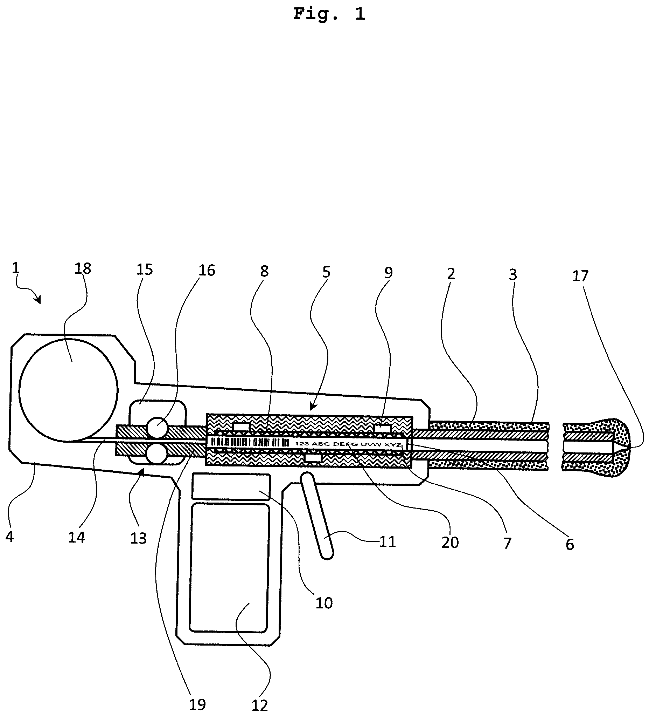

Der vordere bestimmungsgemäß mit seinem distalen Ende voran in die Scheide des Tieres einzuführende Teil der erfindungsgemäßen Vorrichtung 1 von Fig. 1 liegt in Fig. 1 rechts. Er ist ähnlich aufgebaut wie bei klassischen Besamungsspritzen. Er weist ein langes, schlankes, an beiden Stirnseiten offenes Rohr 2 auf, welches vor dem Einführen in die Scheide durch eine nur einmal zu verwendende Besamungshülle 3 bedeckt ist.

Das hintere proximale Ende des Rohres 2 ist mit einem Gehäuse 4 starr verbunden und ragt in dieses hinein. Im Gehäuse 4 befindet sich die längliche, koaxial zum Rohr 2 ausgerichtete Heizeinheit 5. Die Heizeinheit 5 weist einen in ihrer Längsrichtung durchgehenden Hohlraum 6 auf. In dem in Fig. 1 dargestellten betriebsbereiten Zustand der Vorrichtung 1 ist in dem Hohlraum 6 die Paillette 7 angeordnet, welche das letztendlich in den Uterus des Tieres einzubringende Sperma enthält.

Durch die Mantelfläche des länglichen Hohlraums 6 in der Heizeinheit 5 ist die Mantelfläche der Paillette 7 eng umfasst und somit in eng definierter Position gehalten. Bevorzugt möglichst direkt an der Mantelfläche des Hohlraums 6 und damit am Berührungsbereich der Heizeinheit 5 mit der Paillette 7 ist die Heizeinheit 5 mit einem elektrischen Heizwiderstand 8 ausgestattet. Fließt elektrischer Strom durch den Heizwiderstand 8, so erwärmt sich dieser und gibt Wärme an seine Umgebung und somit an die Paillette 7 ab. Im Nahbereich des Heizwiderstandes 8 und der Paillette 7 sind Temperatursensoren 9 angeordnet, welche in Abhängigkeit der an ihnen herrschenden Temperatur ein elektrisches Signal an eine Steuerung 10 liefern. In der Steuerung 10 ist für den Erwärmungsvorgang einer Paillette 7 datentechnisch eine Soll-Heizkurve für den Temperaturverlauf über die Zeit an den Temperatursensoren 9 hinterlegt. Sobald der Erwärmungsvorgang ausgelöst wird, was durch Drücken des Auslösehebels 11 geschehen kann, schickt die Steuerung passend elektrischen Strom durch den Heizwiderstand 8, damit um diese so zu erhitzen, dass der besagte Temperaturverlauf an den Temperatursensoren 9 bestmöglich stattfindet, womit die Paillette 7 in optimaler Weise erwärmt wird. Die erforderliche elektrische Energie wird von einer elektrische Batterie 12, welche idealerweise wiederaufladbar ist, bereitgestellt. (Anstatt eines auf dem Prinzip des ohmschen Widerstandes basierenden Wärmequelle in welcher elektrische Energie in Wärme umgewandelt wird, könnte auch eine auf Mikrowellenstrahlung oder auf Induktion basierendes Prinzip zur Umwandlung von elektrischer Energie in Wärme angewandt werden.)

Wenn die Paillette die Zieltemperatur für die künstliche Besamung erreicht hat, wird diese Temperatur durch den aus den Elementen Heizwiderstand 8, Temperatursensoren 9 und Steuerung 10 gebildeten Regelkreis auf stabiles Halten dieses Werts geregelt. Nun kann jederzeit durch nochmaliges Drücken des Auslösehebels 11 jener Antrieb 13 gestartet werden, welcher letztendlich das in der Paillette 7 enthaltende Sperma in den Uterus des zu besamenden Tieres drängt.

In dem in Fig. 1 skizzierten vorteilhaften Ausführungsbeispiel umfasst der Antrieb 13 einen Kolben 14, welcher als elastisch biegsamer Draht ausgeführt ist, einen Elektromotor 15 und ein Paar von Antriebsrollen 16.

Der vordere Teil des Kolbens 14 ist koaxial zur Paillette 7 auf deren hintere Stirnseite hin ausgerichtet. Wie bei Besamungsspritzen gemäß dem Stand der Technik auch, ist der Durchmesser des Kolbens 14, derart bemessen, dass er in das hintere Stirnende der als Röhrchen ausgebildeten Paillette 7 hineinpasst und bei Bewegung auf die Paillette 7 zu in dieser an einem Verschlusspfropfen zum Anliegen kommt.

Bei in Betrieb befindlichem Antrieb 13 treibt der Elektromotor 15 die an der Mantelfläche des Kolbens mit ihrer Mantelfläche anliegenden, am Gehäuse 4 drehbar gelagerten Antriebsrollen 16, zur Drehung an, sodass sie den Kolben 14 in seiner Längsrichtung in Richtung auf die Paillette 7 verschieben. Sobald die Stirnseite des Kolbens 14 an der Paillette anliegt, verschiebt der Kolben die Paillette 7 aus der Heizeinheit 5 hinaus in das zum Hohlraum 6 der Heizeinheit 5 koaxial liegende Rohr 2, hinein, welches zu diesem Zeitpunkt mit seinem vom Gehäuse 4 abgewandten distalen Längsbereich schon in der für den künstlichen Besamungsvorgang optimalen Tiefe in die Scheide bzw. den Uterus des zu besamenden Tieres ragt. Durch den Kolben 14 wird die Paillette 7 auch im Rohr 2 bis an dessen vorderes (= distales) Ende verschoben, bis die Paillette 7 an der distalen Stirnseite der Besamungshülle 3 anliegt und ihre Bewegung damit gestoppt wird. Durch weitere Bewegung des Kolbens 14 auf die Paillette 7 zu, wird der zuvor erwähnte Verschlusspfropfen in der Paillette 7 in Richtung auf deren vorderes Ende hin - also in die Paillette hinein - verschoben, wodurch durch den sich dort aufbauenden Druck des in der Paillette 7 enthaltenen Spermas ein Ventil geöffnet wird und das Sperma erst aus der Paillette 7 austritt und dann auch durch eine kleine stirnseitige Öffnung 17 in der Besamungshülle 3 und somit bestimmungsgemäß in den Uterus des zu besamenden Tieres gelangt. Wenn Pailletten verwendet werden, die kein derartiges Ventil aufweisen, sondern am vorderen Ende abgeschnitten werden müssen, so kann dieses Abschneiden vor dem Anbringen der Paillette in der Heizvorrichtung 5 von Hand aus erfolgen, oder die Vorrichtung 1 ist mit einer automatischen Schneidevorrichtung ausgestattet, welche die Paillette abschneidet. Diese Schneidevorrichtung kann beispielsweise durch eine kleine, in einem engen Winkelbereich schwenkbare Schneide gebildet sein, auf welche gegebenenfalls ein oder zwei Elektromagneten wirken, um eine Schwenkbewegung anzutreiben. Idealerweise ist diese Schneidvorrichtung zwischen der Heizvorrichtung 5 und dem Rohr 2 angebracht.

Nach diesem Vorgang wird die Vorrichtung 1 von dem Tier weg bewegt, sodass Rohr 2 und Besamungshülle 3 schließlich zur Gänze aus der Scheide des Tieres herauskommen. Der Antrieb 13 wird in Gegenrichtung umgeschaltet, sodass der Kolben 14 wieder eingefahren wird und sein überwiegender Längsbereich auf die Rolle 18 aufgewickelt wird. Idealerweise ist dazu auch die Rolle 18 durch einen Elektromotor zu Drehung antreibbar. Schließlich kommt die Paillette 7 an einem Kolbenführungsteil 19 im Nahbereich der hinteren Stirnseite der Heizeinheit 5 zum Anschlag. Durch weitere Bewegung des Kolbens 14 wird dieser aus der Paillette 7 zurückgezogen und die leere Paillette 7 liegt allein im Hohlraum 6 der Heizeinheit.

Für die Zuführung und die Entnahme der Paillette 7 in bzw. aus der Heizeinheit 5 kann beispielsweise die Heizeinheit normal zu ihrer Längsrichtung aus dem Gehäuse 4 heraus verschiebbar sein, sodass die stirnseitigen Öffnungen des Hohlraums 6 zugänglich werden. Die Verschiebbarkeit der Heizeinheit 5 gegenüber dem Gehäuse 4 kann beispielsweise erreicht werden, indem diese in einem Kanal angeordnet und in Kanallängsrichtung geführt verschiebbar gehalten ist, wobei der Kanal seitlich durch die Vorrichtung hindurch führt. Es können auch mehrere Heizeinheiten in Kanalrichtung nebeneinander angeordnet sein und zu einer Baueinheit verbunden sein und so gemeinsam durch den Kanal verschiebbar sein, wobei immer nur jene Heizeinheit an Kontakte mit Stromversorgung für die Heizung trifft, welche sich in jener Position befindet, bei welcher ihr Hohlraum 6 in der Bewegungslinie des Kolbens 14 liegt. Besagte Baueinheit die mehrere Heizeinheiten hält kann man sich beispielsweise auch wie die Trommel eines Trommelrevolvers vorstellen, wobei in den einzelnen Hohlräumen anstatt einer Patrone jeweils eine Heizeinheit angeordnet ist.

Anstatt die Paillette 7 mit Hilfe eines Kolbens 14 zu bewegen und zu entleeren, könnte sie auch durch Fluiddruck, bevorzugt Gasdruck aus dem Hohlraum 6 hinaus in das Rohr 2 bewegt werden und auch entleert werden. Der Gasdruck müsste dazu von der dem Rohr 2 abgewandten Seite des Hohlraums 6 her durch eine Pumpe oder durch eine sonstige Druckquelle wie z.B. eine CO2-Patrone aufgebaut werden, und die Paillette 7 muss dazu im Hohlraum 6 bzw. im Rohr 2 mit ihrer Mantelfläche dichtend eng an der Innenmantelfläche des Hohlraums 6 bzw. des Rohres anliegen.

Für den nächsten künstlichen Besamungsvorgang brauchen an der Vorrichtung 1 nur die gebrauchten Teile Besamungshülle 3 und Paillette 7 durch neue Teile ausgetauscht zu werden.

In einer bevorzugten Ausführungsform wird die Paillette 7 nach ihrem Entleerungsvorgang nicht durch das Rohr 2 hindurch in den Hohlraum 6 zurückbewegt, sondern bleibt am distalen Ende des Rohres 2 und wird von dort während oder nach dem Entfernen der Besamungshülle 3 über die distale Öffnung des Rohres 2 entfernt. Damit wird weiter Gefahr vermieden, dass die Innenmantelfläche des Rohres 2 mit Sperma aus der Paillette 7 in Kontakt kommt.

Gemäß einer vorteilhaften Weiterentwicklung ist in der Heizeinheit 5 auch ein Lesegerät, typischerweise eine kleine Kamera angebracht, welche Identifizierungsinformation 20, mit der die Paillette 7 versehen ist, ausliest. Die Information kann durch die Steuerung 10 zusammen mit weiteren Informationen für Dokumentationszwecke zum sofortigen oder späteren Auslesen gespeichert werden. Die Identifizierungsinformation 20 kann beispielsweise als Beschriftung als Strichcode und/oder als alphanummerische Zeichenfolge an der Außenmantelfläche der Paillette 7 angebracht sein.

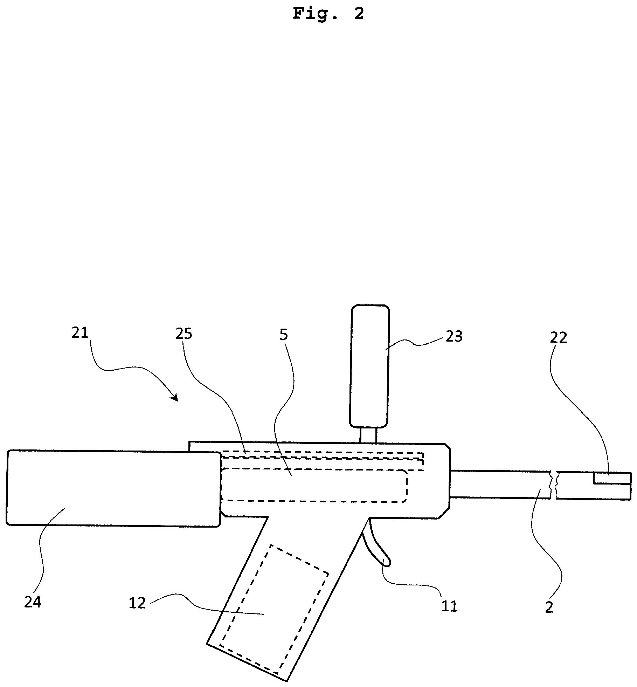

Da die erfindungsgemäße Vorrichtung für die künstliche Besamung ohnedies schon eine elektronische Steuerung, und einen elektrische Energiespeicher enthält sind es vorteilhafte Weiterentwicklungen, an der Vorrichtung weitere funktionelle Baugruppen unterzubringen, welche unter Anwendung von elektronischen bzw. elektromechanischen Bauteilen nützliche Funktionen ausführen können. Der Veranschaulichung derartiger Funktionen dient Fig. 2.

Die beispielhafte erfindungsgemäße Vorrichtung 21 für die künstliche Besamung eines Säugetieres zeigt Teile deren Funktion schon an Hand von Fig. 1 erklärt wurde. Diese Teile sind das Rohr 2, die Heizeinheit 5, der Auslösehebel 11 und die Batterie 12. An weiteren funktionellen Teilen zeigt Fig. 2:
Am vorderen (distalen) Ende des Rohres 2 ist eine optische Aufnahmeeinheit Sensoreinheit 22 angebracht. Diese kann eine auch mit einer Lichtquelle ausgestatte Endoskopkamera 22 sein. Die von dieser Kamera während des Einführens des Rohres aufgenommene Bildinformation kann am Display 23 dargestellt werden. Damit wird es erleichtert, das Rohr 2 in die jeweils richtige Richtung zu bewegen und zu erkennen wann das Rohr 2 passend weit eingeführt ist. Weiters wird es damit auch möglich, ohne das eingangs erwähnte Ertasten der Position des Rohres vom Darm aus unnötig zu machen, was natürlich eine extreme Arbeitserleichterung für den Besamungstechniker darstellt.

Unter Umständen kann auch Information darüber erkannt werden, ob das Tier überhaupt empfängnisfähig ist.

Die Sensoreinheit 22 kann alternativ oder ergänzend zur Endoskopkamera 22 - die im Spektralbereich des sichtbaren Lichtes arbeitet - auch eine NIR-Sensorik sein oder enthalten. Das heißt dass sie eine oder mehrere Lichtquellen - typischerweise Laserdioden - für ein oder mehrere Spektren von nahem Infrarotlicht enthält, sowie Sensoren für auftreffendes Licht aus dem nahen Infrarotspektralbereich. Wie an sich bekannt, kann damit an Hand der Intensität bestimmter einzelner Spektralbereiche im Verhältnis zu bestimmten anderen Spektralbereichen Konzentrationen verschiedener Stoffe wie beispielsweise Hämoglobin, Leukozyten, Wasser im beobachteten Oberflächengewebe erkannt werden. An Hand der Konzentrationen von Hämoglobin und Wasser kann beispielsweise ein Hinweis geliefert werden, ob sich das Tier tatsächlich in der Brunst befinden kann.

Am Display 23 können Messergebnisse und daraus automatisch gezogene Schlüsse angezeigt werden, und es kann auch ein Auswahlmenü für die Wahl weiterer Schritte angeboten werden. Beispielsweise kann angeboten werden, einen Besamungsvorgang abzubrechen, wenn bei der NIR-Spektroskopie festgestellt wurde, dass sich das Tier allem Anschein gar nicht in der Brunst befindet, oder wenn an Hand festgestellter hoher Konzentration von Leukozyten anzunehmen ist, dass das Tier eine Entzündung aufweist.

Am Display 23 können natürlich auch Informationen über den jeweiligen Status von Teilen der Vorrichtung 21 angezeigt werden, sowie Informationen über die jeweils aktuelle enthaltene Paillette.

Am Gehäuse der Vorrichtung 21 kann ein Kühlbehälter 24 lösbar befestigbar sein, welcher ein bewegbares Magazin umfassen kann in welchem sich mehrere Pailletten befinden, wobei durch elektromechanische Komponenten der Vorrichtung 21 eine individuell ausgewählte Paillette an eine Entnahmeschleuse gebracht und in die Heizeinheit 5 transportiert werden kann. Je nachdem ob gefrorenes Sperma oder nur bei Niedrigtemperatur gehaltenes Sperma in den Pailletten transportiert werden soll, kann der Kühlbehälter 24 für die Mitführung von Flüssigstickstoff ausgelegt sein, oder auch nur für das Halten einer nicht höheren Kühltemperatur.

Die Vorrichtung 21 enthält einen mit einer Lichtquelle zusammenwirkenden optischen Sensor 25, durch welchen das Sperma durchleuchtet und optisch daraufhin analysiert wird, wie hoch die Dichte der lebenden Spermienzellen ist.

Die erfindungsgemäßen Vorrichtungen 1, 21 können mit elektronischen Teilen für sogenannte body coupled communication (kurz BCC) ausgestattet sein. Die Teile sind insbesondere Sensoren, welche die Berührung der Vorrichtung durch den Besamungstechniker und/oder die Berührung der Vorrichtung mit dem zu besamenden Tier detektiert und daraufhin mit einem vom Besamungstechniker mitgeführten bzw. am Tier angebrachten Gerät auf elektronischem Weg kommuniziert, wobei der Körper des Besamungstechnikers bzw. des Tieres als elektrischer Leiter für die Übertragung von elektrischen Signalen verwendet wird. Damit kann automatisch erfasst und dokumentiert werden, welcher Besamungstechniker an welchem Tier mit welcher Vorrichtung zur Besamung 1, 21 hantiert hat.

Als Daten können beispielsweise gespeichert werden: persönliche Daten des Besamungstechnikers, Tier-Identifikation, Daten bezüglich dem Tier von welchem das Sperma stammt, Datum, Zeit Ort zu denen die Besamung stattgefunden hat. (Letztere Informationen können typischerweise durch eine passend ausgerüstete Tierohrmarke am Tier festgestellt werden und durch BCC an die Vorrichtung zur Besamung übertragen werden.)

Indem Identifizierungsinformation über das zu besamende Tier und das verwendete Sperma automatisch erfasst wird, kann unter Vernetzung der Vorrichtung 1, 21 in einem Funknetzwerk auch automatisch überprüft werden, ob tatsächlich die richtige Paillette für das richtige Tier angewendet wird. Im positiven Fall kann der Besamungsvorgang automatisch freigegeben werden, im negativen Fall kann der Besamungsvorgang automatisch blockiert werden.

Die Information über die Identität der jeweiligen Paillette braucht nicht unbedingt auf optischem Weg vorliegen und ausgelesen zu werden. Es ist beispielsweise auch möglich, die Pailletten oder den diese enthaltenden Kühlbehälter 24 mit RFID-Chips auszustatten, aus welchen durch eine Leseeinrichtung an der Vorrichtung 1, 21 Information ausgelesen wird.

Am einzuführenden Stab 2 können weitere Sensoren angebracht sein, mittels denen automatisch Informationen darüber gewonnen werden können, ob das Tier tatsächlich empfängnisbereit ist oder nicht. Die Auswertung dieser Informationen kann ganz oder teilweise im Steuerungsteil der Vorrichtung automatisiert erfolgen. Im negativen Fall kann die Besamung rechtzeitig abgebrochen werden, um die jeweilige Paillette nicht zu verbrauchen. Die durch derartige Sensoren zu gewinnenden Informationen können beispielsweise die Temperatur in der Scheide oder am Uterus des Tieres betreffen, oder einen pH-Wert, oder das Vorhandensein von bestimmten Hormonen, oder eine Farbinformation über den Sensor umgebendes Gewebe oder umgebende Flüssigkeit. Ein Ultraschallsensor kann Auskunft über Beschaffenheit des umliegenden Gewebes und auch über die Position des Sensors selbst liefern.

## Patentansprüche

1. Vorrichtung (1, 21) für die künstliche Besamung eines Säugetieres im Zuge dessen Sperma, welches von einem männlichen Zuchttier stammt, durch die Scheide des Tieres hindurch in oder an den Uterus des zu besamenden Tieres eingebracht wird, wobei die Vorrichtung (1, 21) ein Rohr (2) aufweist, welches durch die Scheide des zu besamenden Tieres hindurch an den Uterus bewegbar ist, sowie einen beheizbaren Hohlraum (6) für die Aufnahme einer Paillette (7) welche Sperma enthält, sowie einen Antrieb (13) durch welchen das Sperma aus der Paillette (7) und der uterusseitigen Öffnung des Rohres (2) herausbewegbar ist, wobei die Vorrichtung (1, 21) einen Mechanismus aufweist, mit Hilfe dessen die Paillette (7) aus dem Hohlraum (6) hinaus in das Rohr (2) verschiebbar ist; **dadurch gekennzeichnet, dass** die Vorrichtung (1, 21) einen optischen Sensor (25) für die Messung der Dichte der lebenden Spermienzellen enthält.

2. Vorrichtung (1, 21) nach Anspruch 1, wobei der beheizbare Hohlraum (6) zum proximalen Ende des Rohres (2) hin offen ist.

3. Vorrichtung (1, 21) nach Anspruch 2, wobei ein Kolben (14) von der dem Rohr (2) abgewandten Seite her in den beheizbaren Hohlraum (6) hinein und durch diesen hindurch in das Rohr (2) hinein bewegbar ist.

4. Vorrichtung (1, 21) nach Anspruch 3 wobei der Kolben (14) durch einen elastisch biegsamen Draht gebildet ist, wobei ein Teil des Drahtes an einer Rolle (18) aufgewickelt ist.

5. Vorrichtung (1, 21) nach einem der Ansprüche 1 bis 4, **dadurch gekennzeichnet, dass** der Hohlraum (6) Teil einer Heizeinheit (5) ist, welche relativ zum Gehäuse (4) der Vorrichtung bewegbar ist.

6. Vorrichtung (1, 21) nach Anspruch 5, **dadurch gekennzeichnet, dass** die Heizeinheit normal zur Achse des Rohres (2) bewegbar ist.

7. Vorrichtung (1, 21) nach einem der Ansprüche 1 bis 6, **dadurch gekennzeichnet, dass** sie eine Lesevorrichtung aufweist, welche dazu in der Lage ist, Identifizierungsinformation (20) von der Paillette (7) auszulesen.

8. Vorrichtung (21) nach einem der Ansprüche 1 bis 7, **dadurch gekennzeichnet, dass** am distalen Bereich des Rohres (2) eine Endoskopkamera angebracht ist.

9. Vorrichtung (21) nach einem der Ansprüche 1 bis 8, **dadurch gekennzeichnet, dass** am distalen Bereich des Rohres (2) ein NIR-Sensor angebracht ist.

10. Vorrichtung (21) nach einem der Ansprüche 1 bis 9, **dadurch gekennzeichnet, dass** sie ein Display (23) aufweist, welches von einer zur Vorrichtung (21) gehörenden Steuerung angesteuert wird und dass die Steuerung dazu ausgelegt ist, am Display (23) Statusinformationen bezüglich der Vorrichtung (21) und/oder Informationen bezüglich des Zustandes des Tieres und/oder Statusinformationen bezüglich des aktuellen Besamungsvorganges anzuzeigen.

11. Vorrichtung (21) nach einem der Ansprüche 1 bis 10, **dadurch gekennzeichnet, dass** sie mit einem Kühlbehälter (24) lösbar verbindbar ist, welcher für die Aufnahme mehrere Pailletten (7) ausgelegt ist.

12. Vorrichtung (21) nach einem der Ansprüche 1 bis 11, **dadurch gekennzeichnet, dass** sie zu elektronischer Kommunikation mittels body coupled communication ausgerüstet ist.

13. Vorrichtung (21) nach einem der Ansprüche 1 bis 12, **dadurch gekennzeichnet, dass** am Rohr (2) Sensoren für die Messung von Zustandsgrößen in der Scheide oder am Uterus des zu besamenden Tieres angebracht sind und dass eine in der Vorrichtung (21) enthaltene Steuerung (10) dazu ausgelegt ist, die Messergebnisse automatisch auf die Aussage hin auszuwerten ob das Tier empfängnisbereit ist oder nicht und in weiterer Folge den Besamungsvorgang freizugeben oder abzubrechen.

## Claims

1. Device (1, 21) for the artificial insemination of a mammal, in the course of which the semen, which is derived from a male breeding animal, is introduced through the vagina of the animal, into or onto the uterus of the animal to be inseminated, wherein the device (1, 21) comprises a tube (2), which is movable through the vagina of the animal to be inseminated to the uterus, as well as a heatable cavity (6) for containing a straw (7), which contains semen, as well as a driving mechanism (13), by which the semen is movable out of the straw (7) on the uterus-side opening of the tube (2),
wherein
the device (1, 21) comprises a mechanism, by which the straw (7) can be displaced out of the cavity (6) into the tube (2); **characterized in that**
the device (1, 21) comprises an optical sensor (25) for measuring the density of the live sperm cells.

2. Device (1, 21) according to claim 1, wherein the heatable cavity (6) is open towards the proximal end of the tube (2).

3. Device (1, 21) according to claim 2, wherein a piston (14) is movable, from the side opposite to the tube (2) into the heatable cavity (6) and therethrough into the tube (2).

4. Device (1, 21) according to claim 3, wherein the piston (14) is formed by an elastically pliable wire, wherein a part of the wire is wound up on a reel (18).

5. Device (1, 21) according to one of claims 1 to 4, **characterised in that** the cavity (6) is part of a heating unit (5), which is movable relative to the housing (4) of the device.

6. Device (1, 21) according to claim 5, **characterised in that** the heating unit is movable in a direction normal to the axis of the tube (2).

7. Device (1, 21) according to one of claims 1 to 6, **characterised in that** it comprises a reading device, which is capable to read identifying information (20) from the straw (7).

8. Device (21) according to one of claims 1 to 7, **characterised in that** the distal portion of the tube (2) is provided with an endoscopic camera.

9. Device (21) according to one of claims 1 to 8, **characterised in that** the distal portion of the tube (2) is provided with an NIR sensor.

10. Device (21) according to one of claims 1 to 9, **characterised in that** it comprises a display (23), which is controlled by a control unit, which belongs to the device (21), and **in that** the control unit is designed so that the display (23) displays status information regarding the device (21) and/or information regarding the state of the animal and/or status information regarding the current insemination process.

11. Device (21) according to one of claims 1 to 10, **characterised in that** it can be detachably connected to a cooling container (24), which is designed for receiving a plurality of straws (7).

12. Device (21) according to one of claims 1 to 11, **characterised in that** it is equipped for electronic communication by body coupled communication.

13. Device (21) according to one of claims 1 to 12, **characterised in that** the tube (2) is provided with sensors for measuring state characteristics in the vagina or at the uterus of the animal to be inseminated and **in that** a control unit (10) contained in the device (21) is designed to automatically evaluate the measurement results for a statement as to whether the animal is ready to conceive or not and to subsequently initiate or stop the insemination process.

## Revendications

1. Dispositif (1, 21) d'insémination artificielle d'un mammifère au cours de laquelle du sperme, lequel provient d'un animal reproducteur mâle, est introduit à travers le vagin de l'animal dans ou près de l'utérus de l'animal à inséminer, dans lequel le dispositif (1, 21) présente un tube (2), lequel est déplaçable à travers le vagin de l'animal à inséminer près de l'utérus, ainsi qu'une cavité (6) chauffable pour la réception d'une paillette (7), laquelle contient du sperme, ainsi qu'un entraînement (13) par lequel le sperme peut être sorti de la paillette (7) et de l'ouverture côté utérus du tube (2),
dans lequel le dispositif (1, 21) présente un mécanisme, à l'aide duquel la paillette (7) peut coulisser hors de la cavité (6) dans le tube (2) ; **caractérisé en ce que**
le dispositif (1, 21) contient un capteur optique (25) pour la mesure de la densité des spermatozoïdes vivants.

2. Dispositif (1, 21) selon la revendication 1, dans lequel la cavité (6) chauffable est ouverte vers l'extrémité proximale du tube (2).

3. Dispositif (1, 21) selon la revendication 2, dans lequel un piston (14) est déplaçable du côté opposé au tube (2) dans la cavité (6) chauffable et à travers celle-ci dans le tube (2).

4. Dispositif (1, 21) selon la revendication 3 dans lequel le piston (14) est formé par un fil élastiquement flexible, dans lequel une partie du fil est enroulée sur un rouleau (18).

5. Dispositif (1, 21) selon l'une des revendications 1 à 4, **caractérisé en ce que** la cavité (6) fait partie d'une unité de chauffage (5), laquelle est déplaçable par rapport au boîtier (4) du dispositif.

6. Dispositif (1, 21) selon la revendication 5, **caractérisé en ce que** l'unité de chauffage est déplaçable normalement à l'axe du tube (2).

7. Dispositif (1, 21) selon l'une des revendications 1 à 6, **caractérisé en ce qu'**il présente un dispositif de lecture, lequel est en mesure de lire une information d'identification (20) de la paillette (7).

8. Dispositif (21) selon l'une des revendications 1 à 7, **caractérisé en ce qu'**une caméra endoscopique est montée sur la zone distale du tube (2).

9. Dispositif (21) selon l'une des revendications 1 à 8, **caractérisé en ce qu'**un capteur NIR est monté sur l'extrémité distale du tube (2).

10. Dispositif (21) selon l'une des revendications 1 à 9, **caractérisé en ce qu'**il présente un écran (23), lequel est commandé par une commande appartenant au dispositif (21) et que la commande est conçue pour afficher à l'écran (23) des informations de statut relatives au dispositif (21) et/ou des informations relatives à l'état de l'animal et/ou des informations de statut relatives à l'opération d'insémination actuelle.

11. Dispositif (21) selon l'une des revendications 1 à 10, **caractérisé en ce qu'**il peut être relié de manière amovible à un récipient de refroidissement (24), lequel est conçu pour la réception de plusieurs paillettes (7).

12. Dispositif (21) selon l'une des revendications 1 à 11, **caractérisé en ce qu'**il est équipé pour la communication électronique par body coupled communication.

13. Dispositif (21) selon l'une des revendications 1 à 12, **caractérisé en ce que** des capteurs pour la mesure de grandeurs d'état dans le vagin ou près de l'utérus de l'animal à inséminer sont montés sur le tube (2) et qu'une commande (10) contenue dans le dispositif (21) est conçue pour évaluer automatiquement les résultats de mesure sur la base de l'affirmation si l'animal est fécond ou pas et conséquemment continuer ou interrompre l'opération d'insémination.
